Europäisches Patentamt

European Patent Office

Office européen des brevets

(11) Publication number: **0 467 365 A2**

# EUROPEAN PATENT APPLICATION

(21) Application number: **91112032.7**

(22) Date of filing: **18.07.91**

(51) Int. Cl.5: **A61K 37/43**, A61K 31/435, A61K 31/415, A61K 31/46

(30) Priority: **19.07.90 US 554654**

(43) Date of publication of application:
**22.01.92 Bulletin 92/04**

(84) Designated Contracting States:
**DE FR GB IT**

(71) Applicant: **E.R. SOUIBB & SONS, INC.**
**P.O.Box 4000**
**Princeton New Jersey 08543-4000(US)**

(72) Inventor: **Chen, Hong-I**
**No. 30, Lane 427, Kong-Yuan Rd.**
**Tainan 700(TW)**
Inventor: **Brading, Alison F.**
**5, Canal Road**
**Thrupp, Oxon(GB)**

(74) Representative: **Vossius & Partner**
**Siebertstrasse 4 P.O. Box 86 07 67**
**W-8000 München 86(DE)**

(54) Use of a 5-hydroxytryptamine-3(5-HT3) receptor antagonist for the preparation of a pharmaceutical composition for treating urinary incontinence.

(57) The use of a 5-hydroxytryptamine-3 (5-HT$_3$) receptor antagonist for the preparation of a pharmaceutical composition for preventing or treating urinary incontinence is disclosed.

EP 0 467 365 A2

The present invention relates to the use of a 5-hydroxytryptamine-3 (5-HT$_3$) receptor antagonist for the preparation of a pharmaceutical composition for preventing or treating urinary incontinence.

5-Hydroxytryptamine (serotonin:5-HT) was discovered a little over 40 years ago by Rapport et al "Serum vasoconstrictor (serotonin) IV isolation and characterization," J. Biol. Chem. 176, 1243-51 (1948) and has been found to increase the activity of various visceral structures. In 1957, Gaddum et al, "Two kinds of tryptamine receptor," Brit. J. Pharmacol., 12, 323-328, showed that two distinct 5-HT effects were observed in the smooth muscle of guinea pig ileum. One was associated with a direct contraction of smooth muscle (D-response), the other appeared to mediate depolarisation of the cholinergic neurons (M-response). There has been a recent upsurge of interest in a classification of 5-HT receptors to replace the M- and D-receptors proposed in 1957 by Gaddum et al, supra. There is now strong evidence that 5-HT receptors can be divided into three types-5-HT$_1$, 5-HT$_2$, and 5-HT$_3$ with further subdivisions of the 5-HT$_1$ type, and possibly of the other types as well (Bradley et al, "Proposals for the classification and nomenclature of functional receptors for 5-hydroxytryptamine," Neuropharmacology, 25, 563-576, 1986).

Although it is known that the mammalian urinary bladder contracts in response to 5-HT (Gyermek, "Cholinergic stimulation and blockade on urinary bladder," Am. J. Physiol. 201, 325-328, 1961; Gyermek, "Action of 5-hydroxytryptamine on the urinary bladder of the dog," Arch. Int. Pharmacodyn., 137, 137-144, 1962; Ambache et al, "Non-cholinergic transmission by post-ganglionic motor neurons in the mammalian bladder," J. Physiol. 210, 761-783, 1970; Tiara, N. "The autonomic pharmacology of the bladder," Ann. Rev. Pharmacol., 12, 197-203, 1972; Saum et al, "The actions of 5-hydroxytryptamine on the urinary bladder and on vesical autonomic ganglian in the cat," J. Pharmacol. Exp. Ther., 185, 70-83, 1973), the mechanism of action of 5-HT and the identification of the receptors in the lower urinary tract of various species is still unclear. It is known that different 5-HT receptors are present in the lower urinary tract of various species (Saxena et al, "Excitatory 5-hydroxytryptamine receptors in the cat urinary bladder are of the M- and 5-HT$_2$-type," J. Autan. Pharmacol., 5, 101-107, 1985; Klarskov et al, "Influence of serotonin on lower urinary tract smooth muscle in vitro," Br. J. Urol., 58, 507-513, 1986; Holt et al, "On the nature of the receptor mediating the action of 5-hydroxytryptamine in potentiating responses of the mouse urinary bladder strip to electrical stimulation," Naunyn-Schmiedeberg's Arch. Pharmacol., 334, 333-340, 1986).

The atropine-resistant component of the parasympathetic nerve stimulated contraction in the bladders of several species has been known for many years (Langley et al, "The innervation of the pelvic and adjoining viscera," J. Physiol., 19, 71-139, 1895; Henderson et al, "The role of acetylcholine in bladder contractile mechanisms and in parasympathetic ganglia," J. Pharmacol. & Exper. Therap., 51, 97-111, 1934; Ambache, "The use and limitations of atropine for pharmacological studies on autonomic effectors," Pharmacol. Rev. 7:467-494, 1955). This has been interpreted to be due to the presence of non-adrenergic non-cholinergic excitatory nerves (Ambache et al, supra, 1970; Burnstock et al, 1972, "Atropine resistant excitation of the urinary bladder: the possibility of transmission via nerves releasing a purine nucleotide," Br. J. Pharmacol., 44, 451-461; Downie et al, 1977, "The contribution of cholinergic postganglionic neurotransmission to contractions of rabbit detrusor," J. Pharmacol. Exp. Ther., 203, 417-425), and there is now considerable evidence that ATP is an excitatory transmitter in the urinary bladder of small mammals. The recently synthesized ATP analogue, $\alpha,\beta$-methylene ATP, which is resistant to hydrolysis and has been shown to activate and then desensitize P$_2$-purinoceptors, not only abolishes ATP-induced contraction but also the atropine-resistant response to excitatory nerve stimulation (Kasakov et al, 1983, "The use of the slowly degradable analog, $\alpha,\beta$-Methylene ATP, to produce desensitization of the P$_2$-purinoceptor: effect on non-adrenergic, non-cholinergic responses of the guinea-pig urinary bladder," Eur. J. Pharmacol., 86, 291-294; Fujii, 1988, "Evidence for adenosine triphosphate as an excitatory transmitter in guinea-pig, rabbit and pig urinary bladder," J. Physiol. 404, 39-52; Brading et al, 1989, "Electrical and mechanical responses of guinea-pig bladder muscle to nerve stimulation," Br. J. Pharmacol., 98, 1083-1090).

Urinary incontinence is a major clinical problem not only in the elderly, but also amongst the general population.

It has now been found that there are 5-hydroxytryptamine-3 (5-HT$_3$) receptors in the rabbit lower urinary tract, and that the contractile response to 5-hydroxytryptamine (5-HT) in the lower urinary tract of the rabbit is mediated by presynaptic stimulation. Furthermore, it has been found that 5-HT produces dose-dependent conditions in the detrusor and urethra of the rabbit and that the 5-HT-induced contraction can be dose-dependently inhibited by 5-HT$_3$ antagonists ("Evidence for the presynaptic action of 5-hydroxytryptamine and the involvement of purinergic innervation in the rabbit lower urinary tract," Hong-I, Chen, to be published in British Journal of Pharmacology).

In accordance with the present invention, the use of a 5-HT$_3$ receptor antagonist is disclosed for the preparation of a pharmaceutical composition for preventing or treating urinary incontinence.

The 5-HT$_3$ receptor antagonists which may be employed in the preparation of the invention include,, but are not limited to, zacopride, 3α-tropanyl-1H-indole-3-carboxylic acid ester (ICS 205930, Sandoz); [endo]-N-(9-methyl-9-azabicyclo-[3,3,1]-non-3-yl)-1-methyl-1H-indazole-3-carboxamide hydrochloride (BRL 43694, Granisetron, Beecham); (1αH,3α,5αH-tropan-3-yl-3,5-dichlorobenzoate (MDL 72222, Merrell Dow); ondansetron; and Glaxo's GR 65,630 (3-[5-methyl-1H-imidazol-4-yl]-1-[1-methyl-1H-indol-3-yl]-1-propanone). Preferred are MDL 72222, ICS 205930 and BRL 43694

According to the present invention, the 5-HT$_3$ antagonist containing a pharmaceutical composition may be administered to mammalian species, such as monkeys, dogs, cats, cows, sheep, rats and humans, and as such may be a conventional systemic dosage form, such as a tablet, capsule, elixir or injectable. The above dosage forms will also include the necessary carrier material, excipient, lubricant, buffer, antibacterial, bulking agent (such as mannitol), anti-oxidants (ascorbic acid or sodium bisulfite) or the like. Oral dosage forms are preferred, although parenteral forms such as intramuscular, intraperitoneal, or intravenous are quite satisfactory as well.

The dose administered must be carefully adjusted according to age, weight and condition of the patient, as well as the route of administration, dosage form and regimen and the desired result.

Thus, for oral administration, a satisfactory result may be obtained employing the 5-HT$_3$ antagonist in an amount within the range of from about 0.01 mg/kg to about 100 mg/kg and preferably from about, 0.1 mg/kg to about 25 mg/kg.

A preferred oral dosage form , such as tablets or capsules, will contain the 5-HT$_3$ antagonist in an amount of from about 0.1 to about 500 mg, preferably from about 2 to about 200 mg, and more preferably from about 25 to about 150 mg.

For parenteral administration, the 5-HT$_3$ antagonist will be employed in an amount within the range of from about 0.005 mg/kg to about 20 mg/kg and preferably from about 0.01 mg/kg to about 1 mg/kg.

The composition described above may be administered in the dosage forms as described above in single or divided doses of one to four times daily. It may be advisable to start a patient on a low dose combination and work up gradually to a high dose combination.

Tablets of various sizes can be prepared, e.g., of about 50 to 700 mg in total weight, containing the active substance in the range described above, with the remainder being a physiologically acceptable carrier of other materials according to accepted pharmaceutical practice. These tablets can, of course, be scored to provide for fractional doses. Gelatin capsules can be similarly formulated.

Liquid formulations can also be prepared by dissolving or suspending the active substance in a conventional liquid vehicle acceptable for pharmaceutical administration so as to provide the desired dosage in one to four teaspoonfuls.

Such dosage forms can be administered to the patient on a regimen of one to four doses per day.

In formulating the composition containing the 5-HT$_3$ antagonist, the active substance, in the amounts described above, are compounded according to accepted pharmaceutical practice with a physiologically acceptable vehicle, carrier, excipient, binder, preservative, stabilizer, flavor, etc., in the particular type of unit dosage form.

Illustrative of the adjuvants which may be incorporated in tablets are the following: a binder such as gum tragacanth, acacia, corn starch or gelatin; an excipient such as dicalcium phosphate or cellulose; a disintegrating agent such as corn starch, potato starch, alginic acid or the like; a lubricant such as stearic acid or magnesium stearate; a sweetening agent such as sucrose, lactose or saccharin; a flavoring agent such as orange, peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets or capsules may be coated with shellac, sugar or both. A syrup of elixir may contain the active compound, water, alcohol or the like as the carrier, glycerol as solubilizer, sucrose as sweetening agent, methyl and propyl parabens as preservatives, a dye and a flavoring such as cherry or orange.

Some of the active substances described above may form commonly known, pharmaceutically acceptable salts such as alkali metal and other common basic salts or acid addition salts, etc. References to the base substances are therefore intended to include those common salts known to be substantially equivalent to the parent compound.

The formulations as described above will be administered for a prolonged period, that is, for as long as the potential for onset of urinary incontinence remains or the symptoms of urinary incontinence continue. Sustained release forms of such formulations which may provide such amounts biweekly, weekly, monthly and the like may also be employed. A dosing period of at least one to two weeks are required to achieve minimal benefit.

All of the accompanying Figures are graphs or charts of test data obtained as described in the working Example 6.

The following Examples represent preferred embodiments of the present invention.

Example 1

An injectable solution for use in treating or preventing urinary incontinence is produced as follows:

| Zacopride | 500 mg |
|---|---|
| Methyl paraben | 5 mg |
| Propyl paraben | 1 mg |
| Sodium chloride | 25 g |
| Water for injection qs. | 5 l. |

The zacopride, preservatives and sodium chloride are dissolved in 3 liters of water for injection and then the volume is brought up to 5 liters. The solution is filtered through a sterile filter and aseptically filled into presterilized vials which are then closed with presterilized rubber closures. Each vial contains 5 mL of solution in a concentration of 100 mg of active ingredient per mL of solution for injection.

The so-prepared injectable solution may be administered to treat or prevent urinary incontinence.

Example 2

Two piece #1 gelatin capsules each containing 250 mg of $1\alpha H,3\alpha,5\alpha H$-tropan-3-yl-3,5-dichlorobenzoate (MDL 72222) are filled with a mixture of the following ingredients:

| MDL 72222 | 250 mg |
|---|---|
| Magnesium stearate | 7 mg |
| USP lactose | 193 mg. |

The resulting capsules are useful in treating or preventing urinary incontinence.

Example 3

A $3\alpha$-tropanyl-1H-indole-3-carboxylic acid ester (ICS 205930) formulation suitable for oral administration for use in inhibiting onset of or treating urinary incontinence is set out below.

1000 tablets each containing 100 mg of ICS 205930 are produced from the following ingredients.

| ICS 205930 | 100 g |
|---|---|
| Corn starch | 50 g |
| Gelatin | 7.5 g |
| Avicel (microcrystalline cellulose) | 25 g |
| Magnesium stearate | 2.5 g |

The ICS 205930 and corn starch are admixed with an aqueous solution of the gelatin. The mixture is dried and ground to a fine powder. The Avicel and then the magnesium stearate are admixed with the granulation. This is then compressed in a tablet to form 1000 tablets each containing 100 mg of active ingredient which is used for inhibiting onset of or treating urinary incontinence.

Examples 4 and 5

By substituting 100 g of Granisetron or ondansetron for the ICS 205930 in Example 3 1000 tablets each containing 100 mg Granisetron or 100 mg ondansetron are produced which are useful in inhibiting onset of or treating urinary incontinence.

Example 6

The following experiments were carried out (1) to investigate the mechanism and the nature of the

4

response to 5-HT in the rabbit lower urinary tract, (2) to test whether non-adrenergic non-cholinergic transmission is involved in the 5-HT-induced contraction, and (3) to identify the 5-HT receptor in the rabbit lower urinary tract by application of several 5-HT antagonists.

## Methods

### Preparation of specimens

Rabbit bladder and urethra were obtained form New Zealand White rabbits of either sex, weighing from 600 g to 2500 g. These were stunned by a blow to the neck and exsanguinated. The specimens were placed in oxygenated Krebs solution and strip preparation was performed immediately. A longitudinal cut was made from the anterior wall of the urethra up through the bladder neck to the bladder dome. The mucosa was then dissected free from the bladder and urethral muscles. Strips were cut from the anterior wall, and the posterior wall of the bladders from both sexes. The urethral strips were made in either a longitudinal or transverse direction. All strips of lower urinary tract smooth muscle measured approximately 8 mm x 1 mm x 1 mm unstretched. An operating microscope was used to ensure that there was good longitudinal alignment of the muscle bundles within a strip.

### Tension recording and stimulation

Fine silk ligatures were tied to each end of the strip which was then mounted between platinum ring electrodes 1 cm apart in a specially constructed Perspex organ bath. The organ bath had a capacity of 0.2 mLs and was continuously perfused with warmed (35-37° C) Krebs solution at a flow rate of 1 mL/minute. Six organ baths mounted in parallel allowed six strips to be studies simultaneously. Initially, the strips were allowed to equilibrate for at least one hour, after a resting tension of 1 gm weight had been applied to each strip. Tension was measured isometrically using Pioden UF1 transducers and recorded on a Watanabe multichannel pen recorder after amplification.

Activation of intrinsic nerves was achieved by electrical field stimulation by pulses with the following parameters: 50 volts, 0.05 msec in width, 5 sec trains at varying frequency. Successive trains of stimuli were given at least 5 minutes after the previous contraction had returned to baseline. After each drug induced response recovery periods of 10-30 minutes were allowed before further drug application. Drugs and different solutions were applied by dipping the ends of the feeder tubes into the appropriate solutions. This allowed accurately timed exposures of the tissues to different solutions, and by following the bubbles introduced when the solution was changed the instant of tissue contact was recorded.

At the commencement of each experiment, the contractile response of the strips to a 2-minute application of 126 mM KCl was obtained and subsequent responses were recorded as a percentage of the control response rather than in gms of tension. This dose of KCl produced a near maximal contraction.

### Drugs

The following drugs were used: 5-Hydroxytryptamine creatinine sulphate complex (5-HT), Adenosine 5'-Triphosphate (ATP), Tetrodotoxin (TTX), $\alpha,\beta$-Methyleneadenosine 5'-Triphosphate ($\alpha,\beta$-methylene ATP), Hexamethonium bromide, Prazosin hydrochloride (all these drugs were obtained from Sigma). Atropine sulphate (B.D.H.), Methysergide bimaleate and ICS 205930 (3$\alpha$-tropanyl-1H-indole-3-carboxylic acid ester, Sandoz), Phentolamine mesylate (Ciba), BRL 43694 (Granisetron, [endo]N-(9-methyl-9-azabicyclo-[3,3,1]-non-3-yl)-1-methyl-1H-indazole-3-carboxamide hydrochloride, Beecham), Ketanserine tartrate (Janssen), MDL 72222 (1$\alpha$H,3$\alpha$,5$\alpha$H-tropan-3-yl-3,5-dichlorobenzoate, Merrell Dow), Metitepine monomethanesulfonate (Roche). Drugs were, where possible, dissolved in distilled water to make a concentrated stock solution, these were refrigerated until needed. MDL 72222 was made up as a stock solution of $10^{-3}$M with 75% ethanol, prazosin was dissolved in DMA (1:9, B.D.H., NN-dimethylacetamide) as a stock solution of $10^{-3}$M, and diluted appropriately with Krebs solution; 5-Hydroxytryptamine, ATP and $\alpha,\beta$-methylene ATP, were kept frozen. Drug concentrations are reported as the final bath values. The Krebs solution used had the following composition (mM): NaCl 120.0, KCl 5.9, CaCl$_2$ 2.5, MgCl$_2$ 1.2, NaHCO$_3$ 15.4, NaH$_2$PO$_4$ 1.0, Glucose 11.5. All solutions were equilibrated with 97% O$_2$, 3% CO$_2$, pH 7.4 at 35-37° C. High K+ solution (126 mM) was prepared by replacing NaCl with an equimolar amount of KCl in normal Krebs solution.

### Statistical analysis

Student's t-test was used to compare differences in responses between the control and experimental curves. A probability level of p < 0.05 was accepted as significant. When appropriate, results are presented as means ± standard error of the mean. $pA_2$, which was calculated from a Schild plot, is defined as the negative logarithm of the molar concentration of antagonist required to produce an agonist dose ratio equal to 2. $pA_2$ values were used to express the potency of each competitive antagonist.

All points on each graph are means of at least 6 muscle strips taken from 3 different rabbits.

Results

Effect of 5-HT and 5-HT antagonists

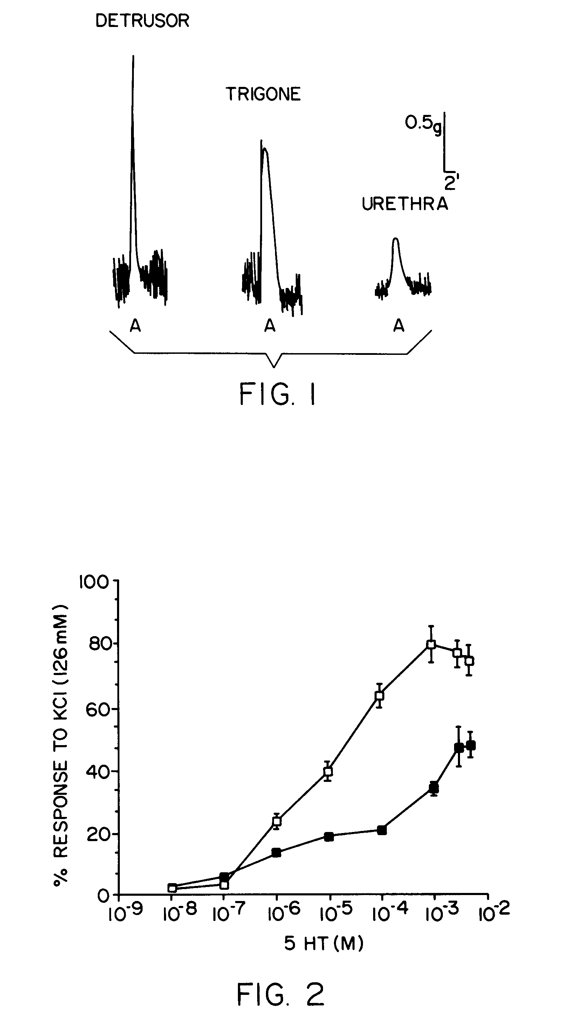

A 30-second application of 5-HT produced dose-dependent contraction in the rabbit bladder ($10^{-8}$ to $10^{-3}$M) and in the urethra ($10^{-6}$ to $10^{-3}$M). A bigger and sharper contraction was seen in the detrusor than in the trigone and urethra (Figures 1 and 2). Biphasic responses which consisted of a small relaxation followed by contraction were present in some rabbit urethra experiments. When higher concentration ($\geq$ $10^{-5}$M) or longer superfusion ($\geq$ 30 seconds) was used, the responses to 5-HT were easily desensitized (n = 9). Thus, only one dosage of the higher concentration of 5-HT was applied for 30-seconds in any one experiment. There was no difference in the response to 5-HT between anterior and posterior detrusor, or between the sexes.

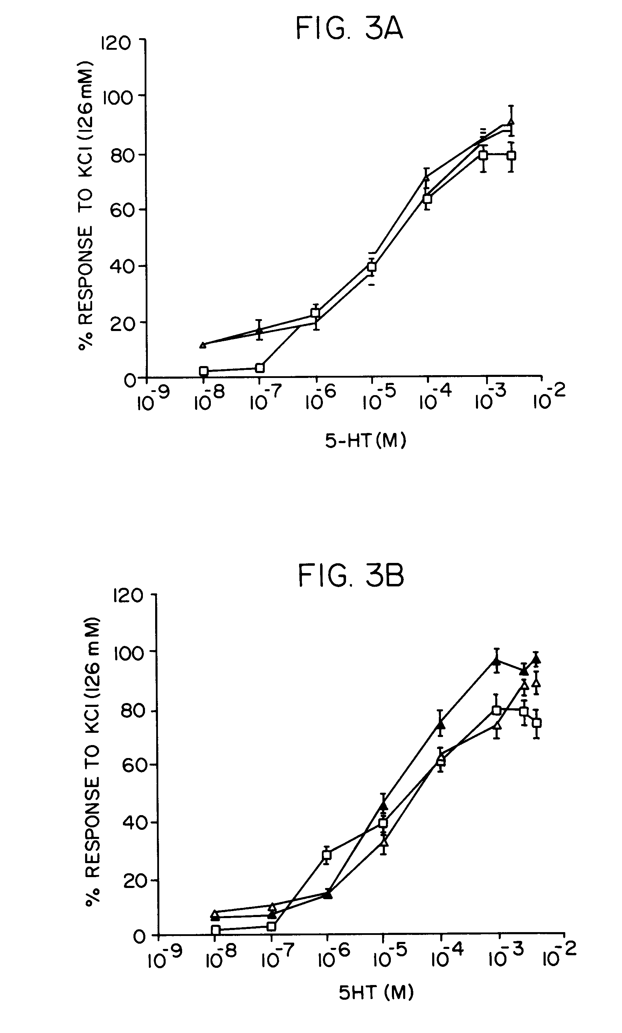

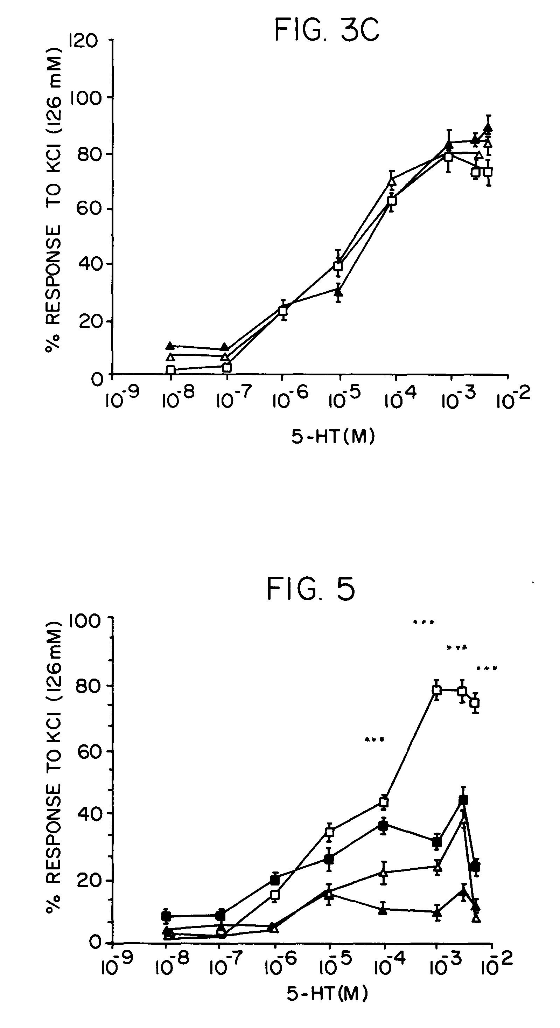

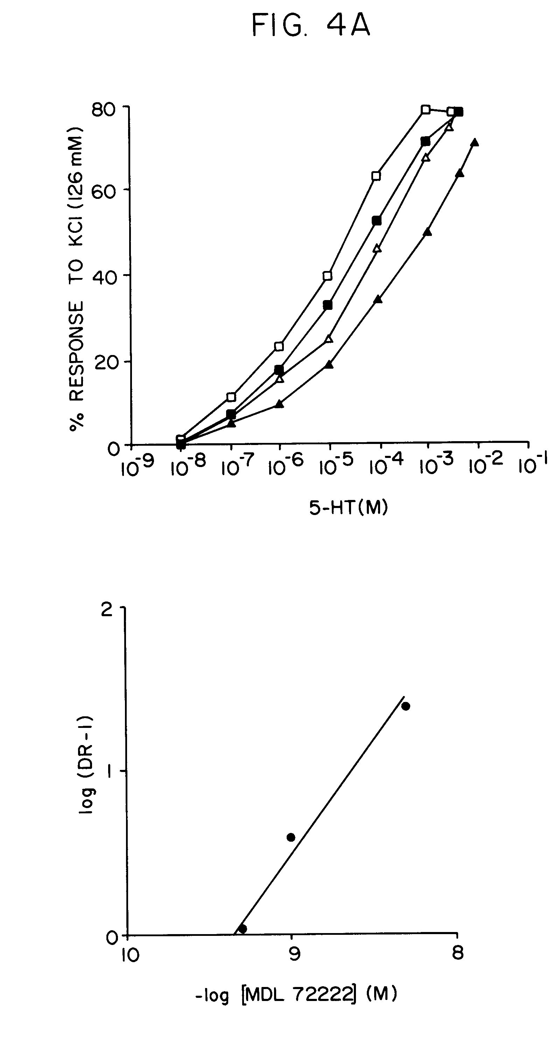

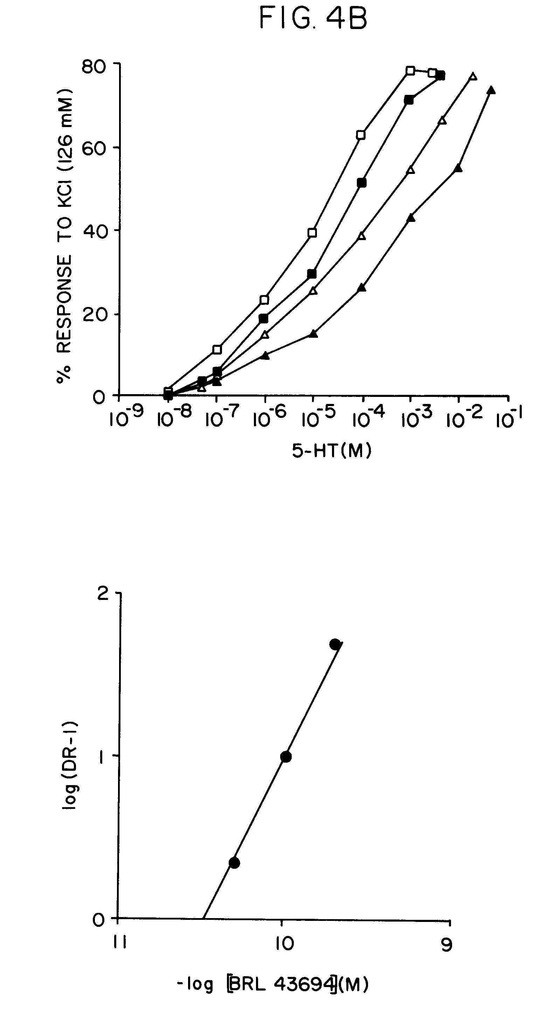

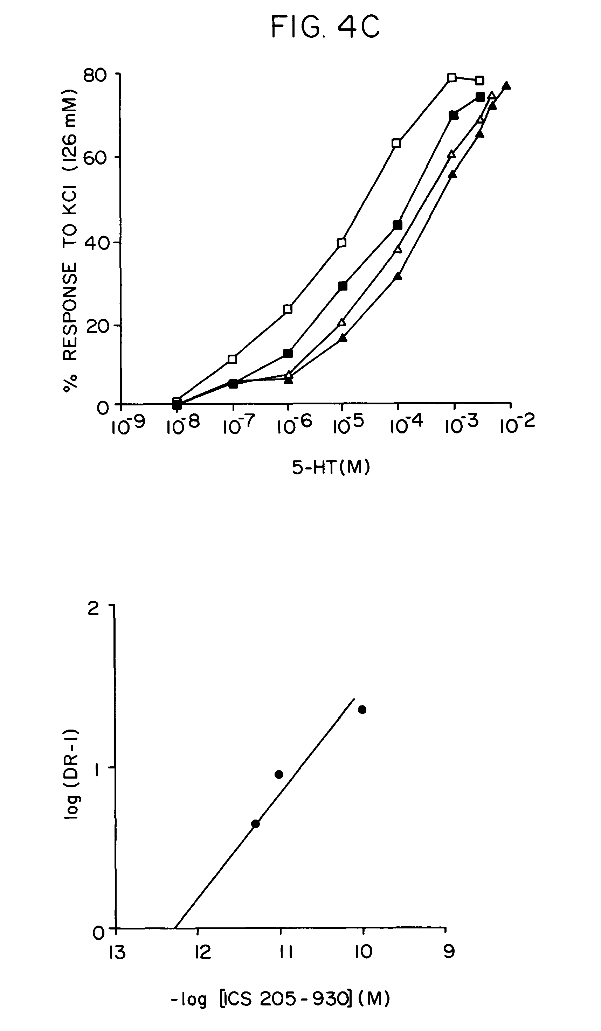

Putative 5-HT antagonists were tested. Ketanserine ($10^{-7}$ to $10^{-6}$M), methysergide ($10^{-8}$ to $10^{-7}$M) and metitepine ($10^{-8}$ to $10^{-7}$M) had no effect on the contractile response to 5-HT (Figures 3a,b,c). However, BRL 43694 ($5 \times 10^{-11}$ to $2 \times 10^{10}$M), ICS 205930 ($5 \times 10^{-12}$ to $1 \times 10^{-10}$M), and, MDL 72222 ($5 \times 10^{-10}$ to $5 \times 10^{-9}$M), after 30 minutes exposure all strongly and competitively antagonized the 5-HT-induced contraction in the rabbit bladder strips (Figure 4). The $pA_2$ values were: for MDL 72222, 9.3 ± 0.04 (n = 9), for BRL 43694, 10.5 ± 0.01 (n = 6) and for ICS 205930, 12.5 ± 0.36 (n = 8).

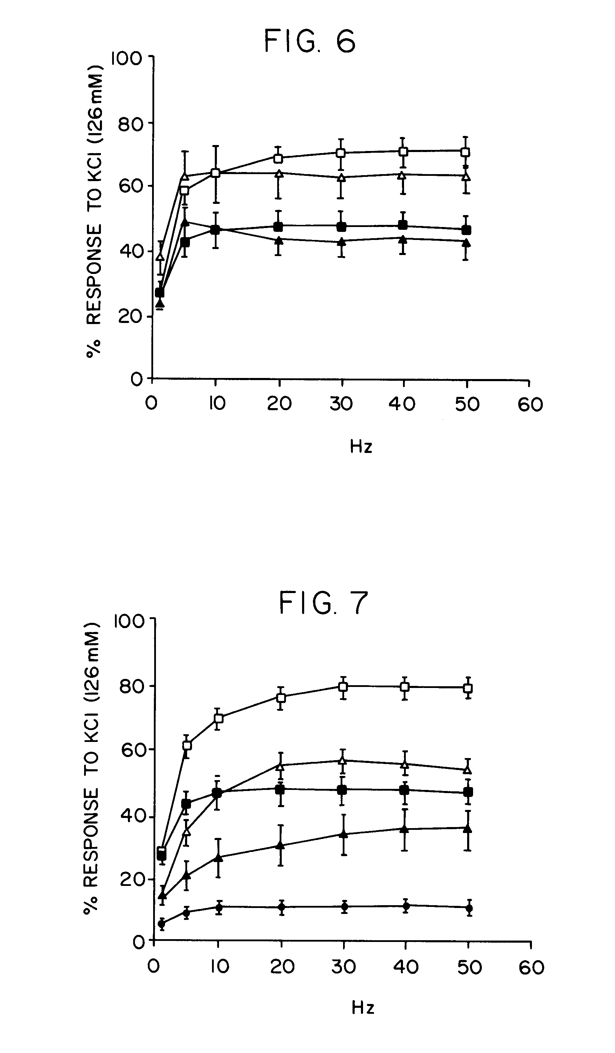

Atropine ($10^{-7}$M) and $\alpha,\beta$-methylene ATP ($10^{-6}$M) both partially inhibited the 5-HT contractions. When applied together, they produced more inhibition of contractile response to 5-HT than either alone (Figure 5). There was a small atropine and $\alpha,\beta$-methylene ATP-resistant component of 5-HT-induced contraction in the bladder strips, which was not abolished by the $5-HT_1$ antagonist metitepine, the $5-HT_2$ antagonists ketanserine and methysergide, or by the $5-HT_3$ antagonists MDL 72222, ICS 205930 and BRL 43694.

TTX ($1.6 \times 10^{-6}$M), hexamethonium ($10^{-6}$M), phentolamine ($10^{-6}$M) and prazosin ($10^{-6}$M) did not affect the 5-HT-induced contraction of the rabbit bladder.

Electrical field stimulation

Nerve-mediated responses of the rabbit bladder were studied using electrical impulses at frequencies of 1, 5, 10, 20, 30, 40 and 50 HZ to selectively stimulate the intramural nerves. The contractile response increased at frequencies up to 30 Hz, and then reached a plateau. There was no difference in the response to field stimulation between male and female rabbit bladder. Abolition of the responses by TTX ($1.6 \times 10^{-6}$M) was complete at all frequencies in the experiments. 20 minute pretreatment of atropine caused dose-dependent ($10^{-8}$ to $10^{-6}$M) inhibition of the response to field stimulation in the rabbit bladder preparations with a maximum at high frequencies with an inhibition of 40% (Figure 6). The residual response was termed the atropine-resistant component. $\alpha,\beta$-methylene ATP caused a dose-dependent inhibition at all frequencies. At $10^{-6}$M, about 35% inhibition was seen. Together atropine ($10^{-7}$M) and $\alpha,\beta$-methylene ATP ($10^{-6}$M) produced a greater inhibition of the contractile response to field stimulation than either alone (Figure 7). However, the $5-HT_3$ antagonist MDL 72222 ($10^{-7}$M) showed no effect on the contraction to field stimulation of the strips.

Figure 1 is a graph which shows contractile response to 5-hydroxytryptamine (5-HT) $10^{-3}$M applied for 30 second to the rabbit lower urinary tract smooth muscles. A bigger and sharper contraction was present in the detrusor than in the trigone and urethra preparations.

Figure 2 is a graph which shows dose-dependent responses to 5-HT in anterior detrusor of normal female rabbit (□) and urethra (■). Points represent means of 15-20 experiments of detrusor and 9-12 experiments of urethra; vertical bars show s.e.mean. Desensitization of the contractile response to 5-HT with higher concentrations ($\geq 10^{-3}$M) can be seen for the detrusor experiments.

Figures 3 a, b and c are graphs which show effect of ketanserine (a, n = 10), methysergide (b, n = 11) or metitepine (c, n = 9) on the contractile responses to 5-HT of anterior detrusor of female rabbits. Results showed no significant effect on the contractile response to 5-HT. △ represents $10^{-7}$M ketanserine, $10^{-8}$M methysergide or metitepine; ▲ represents $10^{-6}$M ketanserine, $10^{-7}$M methysergide or metitepine; Vertical bars indicate s.e.mean.

Figures 4 a, b and c are graphs which show effect of 5-HT antagonists MDL 72222, BRL 43694 and ICS 205930 on rabbit detrusor preparations. (a) MDL 72222 5 x $10^{-10}$M (■), $10^{-9}$M (△) and 5 x $10^{-9}$M (▲) caused dose-dependent inhibition, the response curves shifted to right of the control (□). The $pA_2$ value from the Schild plot was 9.3 ± 0.04. (b) BRL 43694 5 x $10^{-11}$M (■), $10^{-10}$M (△) and 2 x $10^{-10}$M (▲) also caused dose-dependent inhibition. The $pA_2$ value was 10.5 ± 0.01 (n = 6). (c) ICS 205-930 5 x $10^{-12}$M (■), $10^{-11}$M (△) and $10^{-10}$M (▲) showed most potent antagonistic effect; its $pA_2$ value was 12.5 ± 0.36 (n = 8). Each point represents the mean of at least 9 experiments from 6 animals.

Figure 5 is a graph which shows effect of atropine and $\alpha,\beta$-methylene ATP on the contractile response to 5-HT in anterior detrusor of male rabbits. A combination of atropine $10^{-6}$M and $\alpha,\beta$-methylene ATP $10^{-6}$M (▲, n = 12) caused greater inhibition than either atropine (■, $10^{-6}$M, n = 12) or $\alpha,\beta$-methylene (△, $10^{-6}$M, n = 12) alone. Vertical bars show s.e.mean; $^{***}$p ≤0.001 compared to control (□).

Figure 6 is a graph which shows effect of atropine on the contractile response to electrical field stimulation in the posterior detrusor of male rabbits. Results showed that atropine ($10^{-8}$M, △, n = 9; $10^{-7}$M, ■, n = 9; $10^{-6}$M, ▲, n = 12) only partially inhibited the response to field stimulation. Atropine was least effective at 1 Hz, at higher frequencies the blockade was more effective, but approximately 60% of the control response still persisted at 5-50 Hz. Vertical bars show s.e.mean. Stimulus strength 50 volts; stimulus duration 0.05 msec. 5-second train of impulses.

Figure 7 is a graph which shows effects of atropine and $\alpha,\beta$-methylene ATP on the contractile response to electrical field stimulation in the posterior detrusor of male rabbits. Results showed that a combination of $10^{-7}$M atropine and $10^{-6}$M $\alpha,\beta$-methylene ATP (●, n = 9) caused greater inhibition than either atropine (■, $10^{-7}$M, n = 9) or $\alpha,\beta$-methylene ATP (△, $10^{-6}$M, n = 12; ▲, $10^{-5}$M, n = 10) alone; control (□). Vertical bars show s.e.mean. Stimulus strength 50 volts; stimulus duration 0.05 msec. 5-second train of impulses.

Discussion

The contractile response of the bladder and urethra to 5-HT are complicated, and may involve more than one type of receptor (Cohen, "5-Hydroxytryptamine and non-vascular smooth muscle contraction and relaxation," in The peripheral action of 5-hydroxytryptamine, ed. Forzard, J.R. Chapter 9, pp. 201-219, Oxford, New York, Tokyo: Oxford University Press, 1989). The responses could be caused either by direct effects on the smooth muscle cells, or indirectly via effects on the autonomic innervation of these organs. Both actions have been implicated in the effects of 5-HT on cat bladder (Saxena et al, 1985, supra), but the majority of the work in this field implicates indirect actions as being of major importance, and the ability of 5-HT to stimualate ganglion cells in other tissues, for instance in the enteric nervous system, is well recognized (Gaddum, et al, 1957, supra; Drakontides et al, 1968, "5-Hydroxytryptamine receptors in the mouse duodenum," Br. J. Pharmacol., 33, 480-492; Costa et al, 1979, "The sites of action of 5-HT in nerve-muscle preparation from the guinea-pig small intestine and colon," Br. J. Pharmacol., 65, 237-248; Jin et al, 1989, "Myenteric 5-HT-containing neurones activate the descending cholinergic excitatory pathway to the circular muscle of guinea-pig ileum," Br. J. Pharmacol., 98, 982-988), and there is evidence that 5-HT may actually be one of the neurotransmitters to ganglion cells (Richardson et al, 1985, "Identification of serotonin M-receptor subtypes and their specific blockade by a new class of drugs," Nature, 316, 126-131; Bradley et al, 1986, supra). Since urinary tract smooth muscles may receive excitatory innervation from both parasympathetic and sympathetic pathways, it has been of interest to determine which of these autonomic pathways was involved in the response to 5-HT. Experimental evidence strongly implicates effects via the parasympathetic pathway in all species studied (cat: Saum et al, 1973 supra; dog: Gyermek, 1962, supra; frog; Hirai et al, 1980, "Presynaptic regulation of the release of acetylcholine by 5-hydroxytryptamine," Br. J. Pharmacol. 70, 499-500; rat: Aas, 1983, "Serotonin induced release of acetylcholine from neurons in the bronchial smooth muscle of the rat," Acta Physiol. Scand. 117, 477-480), the drug thought to be activating ganglion cells which result in the release of acetylcholine onto the smooth muscle cells. Results described herein, also suggest the involvement of the parasympathetic pathway in the effects of 5-HT on the rabbit urinary tract smooth muscles, since atropine dose-dependently inhibited the responses. However, there was a clear atropine resistant component of the 5-HT response in this species.

An atropine resistance component of the parasympathetic innervation has long been recognised in most species, and ATP has been implicated as a second excitatory transmitter (Burnstock et al, 1972, supra; Fujii, 1988, supra, Brading et al, 1989, supra). The ATP analogue $\alpha,\beta$-methylene ATP which has been shown to activate and desensitize $P_2$-purinoceptors, not only abolishes ATP-induced contraction, but also the atropine-resistant responses to excitatory nerve stimulation (Kasakov et al, 1983, supra). It was therefore of interest to investigate the atropine resistant component of the 5-HT response in the rabbit bladder, and to compare it to the atropine resistant response to excitatory nerve stimulation. The present experiment revealed that after desensitization of the $P_2$-purinoceptors with $\alpha,\beta$-methylene ATP, the atropine-resistant component of the 5-HT response was inhibited, and that a combination of atropine and desensitization of the $P_2$-purinoceptors abolished all but a small component of the 5-HT response. A very similar pattern of blockade was seen with transmural stimulation of the excitatory nerves. In addition, the 5-HT responses were not blocked by phentolamine, prazosin or hexamethonium. This strongly suggests that 5-HT is acting to release the excitatory transmitters from the intrinsic nerves in the rabbit, and that an adrenergic mechanism is not involved.

TTX, which is known to block $Na^+$ channels in the nerve axons, and which completely blocks the excitatory effect of transmural nerve stimulation in the rabbit had no effect on the 5-HT-induced contraction in the present study. A possible explanation is that 5-HT may act on receptors on the nerve terminal, to induce membrane depolarization and transmitter release. Shuster et al, "Cyclic AMP-dependent protein kinease closes the serotonin-sensitive $K^+$ channels of aplysia sensory neurones in cell-free membrane patches," Nature, 313, 392-395 (1985), have demonstrated that 5-HT can close cAMP-sensitive $K^+$ channels in aplysia neurones, which would lead to depolarization, and Higashi et al "5-Hydroxytryptamine receptors of visceral primary afferent neurones on rabbit nodose ganglia," J. Physiol. 323, 543-567, (1982) have demonstrated that 5-HT can open $Na^+$ channels in the nodose ganglion cells of the rabbit. It has also been suggested that excitation of 5-HT receptors on nerve terminals may release acetylcholine in rat bronchi (Aas, 1983, supra), and rabbit heart (Fozard, "Characteristics of the excitatory 5-HT receptor on the cholinergic nerves of rabbit heart," Proceedings of IUPHAR Ninth International Congress of Pharmacology, London, July 1984, and Holt et al, (1986, supra), have shown that 5-HT could enhance the responses to excitatory (cholinergic) innervation presynaptically in the mouse bladder.

The classes of 5-HT receptors involved in the physiological effects have been extensively studied by means of radio-ligand binding studies, and classical pharmacological organ-bath techniques. At least three classes of receptor have been demonstrated (Bradley et al, 1986, supra), one of which, the $5-HT_3$-receptor, has clearly been identified in the peripheral nervous system (Fozard, 1984a, "Neuronal 5-HT receptors in the periphery," Neuropharmacology, 23, 1473-1486; Richardson et al, 1986, supra; Fake et al, 1987, "BAL 43694: a potent and novel $5-HT_3$ receptor antagonist," Br. J. Pharmacol., 91, 335P; Sanger, 1987, "Increased gut cholinergic activity and antagonism of 5-hydroxytryptamine M-receptors by BRL 24924: potential clinical importance of BRL 24924," Br. J. Pharmacol., 91, 47-87). In the present study, the $5-HT_3$-receptor antagonists ICS 205930, BRL 43695 and MDL 72222 were all effective at blocking the excitatory responses of the bladder, whilst the $5-HT_1$ and $5-HT_2$-antagonists were ineffective. Thus in the rabbit $5-HT_3$-receptors mediate the responses which are caused by release of ATP and acetylcholine from the nerve terminals. There was, however, a residual contractile response to 5-HT, which was not blocked by the $5-HT_1$, $5-HT_2$ or $5-HT_3$ receptors, suggesting that there is another 5-HT-receptor which is having a small effect either directly on the smooth muscle, or indirectly through an effect on other nerve endings. The fact that there is also a residual response to transmural nerve stimulation in the presence of atropine and desensitization of the $P_2$-purinoceptors, which is blocked-by TTX, suggests that in this species a minor component of excitation may exist involving a third transmitter, and that 5-HT may be releasing this from nerve terminals through a non-typical $5-HT_3$ receptor.

In summary, the involvement of release of acetylcholine and a non-adrenergic non-cholinergic neurotransmitter in the 5-HT-induced contraction is strongly suggested by the experiments. The results also identified the receptor involved in the rabbit lower urinary tract as the $5-HT_3$ receptor.

**Claims**

1. Use of a 5-hydroxytryptamine-3($5-HT_3$) receptor antagonist for the preparation of a pharmaceutical composition for preventing or treating urinary incontinence.

2. The use as defined in Claim 1 for the treatment of urinary incontinence.

3. The use as defined in Claim 1 for the prevention of urinary incontinence.

4. The use as defined in Claim 1 wherein the $5-HT_3$ receptor antagonist is zacopride; $3\alpha$-tropanyl-1H-indole-3-carboxylic acid ester; [endo]N-(9-methyl-9-azabicyclo-[3,3,1]-non-3-yl)-1-methyl-1H-indazole-3-carboxamide; $1\alpha H,3\alpha,5\alpha H$-tropan-3-yl-3,5-dichlorobenzoate; ondansetron; or 3-(5-methyl-1H-imidazol-4-yl)-1-(1-methyl-1H-indol-3yl)-1-propanone.

5. The use as defined in Claim 4 wherein the 5-HT$_3$ receptor antagonist is 3$\alpha$-tropanyl-1H-indole-3-carboxylic acid ester.

6. The use as defined in Claim 4 wherein the 5-HT$_3$ receptor antagonist is [endo]N-(9-methyl-9-azabicyclo-[3,3,1]-non-3-yl)-1-methyl-1H-indazole-3-carboxamide.

7. The use as defined in Claim 4 wherein the 5-HT$_3$ receptor antagonist is 1$\alpha$H,3$\alpha$,5$\alpha$H-tropan-3-yl-3,5-dichlorobenzoate.

8. The use as defined in Claim 1 wherein said 5-HT$_3$ receptor antagonist is adapted to the administration of single or divided doses of from about 0.1 to about 500 mg/one to four times daily.

9. The use as defined in Claim 1 wherein the 5-HT$_3$ antagonist contains a composition adapted to oral or parenteral administration.

FIG. I

FIG. 2

FIG. 3A

FIG. 3B

FIG. 3C

FIG. 5

# FIG. 4A

# FIG. 4B

# FIG. 4C

FIG. 6

FIG. 7